# EUROPÄISCHE PATENTSCHRIFT

(11) **EP 1 868 976 B1**
(45) Veröffentlichungstag und Bekanntmachung des Hinweises auf die Patenterteilung: **30.12.2015**
(21) Anmeldenummer: 06724048.1
(22) Anmeldetag: 05.04.2006
(51) Int. Cl.: C07C 37/00, C07C 37/68, C07C 37/86, C07C 37/74

(54) **VERFAHREN ZUR HERSTELLUNG VON CARDANOL (I)**
METHOD FOR PRODUCING CARDANOL (I)
PROCEDE POUR PREPARER DU CARDANOL (I)

(30) Priorität: 14.04.2005 DE 102005017126
(43) Veröffentlichungstag der Anmeldung: 26.12.2007
(73) Patentinhaber: Cognis IP Management GmbH, 40589 Düsseldorf (DE)
(72) Erfinder: SATO, Setsuo, CEP-12328-190 Jacarei, SP (BR); BUENO DE ALMEIDA, Wanderson, CEP-12246-300 São José dos Campos, SP (BR); FERREIRA FILHO, Arnaldo, CEP-12322-380 Jacarei, SP (BR); BUENO, Ramiro, Carielo, CEP-12328-390 Jacarei, SP (BR)
(86) Internationale Anmeldenummer: PCT/EP2006/003100
(87) Internationale Veröffentlichungsnummer: WO 2006/108545

(56) Entgegenhaltungen:
- GB-A- 2 066 820
- GB-A- 2 066 820
- GB-A- 2 152 925
- GB-A- 2 262 525
- US-A- 4 352 944

## Beschreibung

### Gebiet der Erfindung

Die Erfindung betrifft ein verbessertes Verfahren zur Herstellung von Cardanol aus CNSL. Das Verfahren zeichnet sich dadurch aus, daß das so erhältliche Cardanol weniger Nebenprodukte aufweist, als das übliche kommerziell erhältliche Cardanol.

### Stand der Technik

Phenalkamine sind eine noch relativ junge Klasse von Epoxidharzhärtern. Es handelt sich dabei um Umsetzungsprodukte (Kondensationsprodukte) von Cardanol (I), das chemisch ein C₁₅-Alkylphenol darstellt und ein Hauptbestandteil des aus den Schalen von Cashew-Nüssen zugänglichen Öls (des sogenannten CNSL = cashew nut shell liquid) ist, mit aliphatischen (primären bzw. sekundären) Aminen und Formaldehyd.

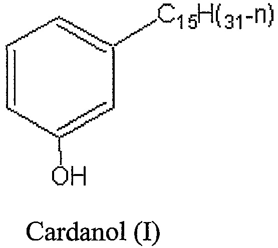

Bezüglich der Klasse der Phenalkamine sei auf folgende Publikation verwiesen: Zhishen Dai et al., "Phenalkamines: Multipurpose Epoxy Curing Agent"; Cardolite Corporation, Newark, New Jersey, USA; Reprint SPI-ERF Conference, September 1994.

Bekanntlich enthält rohes CNSL überwiegend eine Verbindung, die als Anacardic acid (II) bezeichnet wird. Bei der Destillation von CNSL in Gegenwart von Säure erhält man eine Zusammensetzung, die überwiegend Cardanol enthält, als Nebenprodukt jedoch Cardol (III). Vergleiche hierzu zum Beispiel US-B-6,262,148 und US-B-6,229,054. Dies steht im Einklang mit Untersuchungen der Anmelderin, wonach bei der Destillation von rohem CNSL eine Zusammensetzung erhalten wird, die überwiegend Cardanol, als Nebenprodukt jedoch Cardol sowie untergeordnete Mengen an 2-Methylcardol und Anacardic acid enthält.

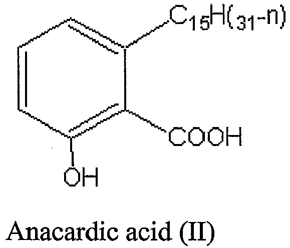

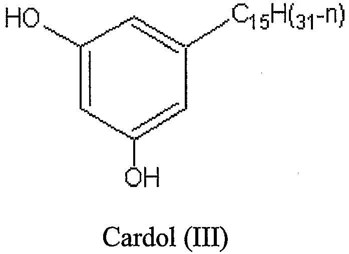

Das auf diese Weise erhaltene Cardanol/Cardol-Gemisch hat im wesentlichen drei technische Nachteile:
- Bei seiner Herstellung durch Destillation aus dem rohen CNSL treten Wertverluste auf, da bei diesem Vorgang ein Teil des Cardanols durch Polymerisation verlorengeht, so daß letztendlich die Cardanol-Ausbeute im Destillat nur 50-60% beträgt.
- Das zunächst leicht gelblich gefärbte Cardanol/Cardol-Gemisch verändert sich bei Lagerung: es kommt rasch zu einer Braunfärbung. Diese unerwünschten Farbveränderungen werden auf die Anwesenheit von Cardol zurückgeführt.
- Auch Folgeprodukte des Cardanol/Cardol-Gemisches zeigen bei Lagerung die geschilderten unerwünschten Farbveränderungen.

Es ist vorgeschlagen worden, die Farbstabilität von Cardanol/Cardol-Gemischen dadurch zu verbessern, indem man den Gehalt an Cardol durch ganz spezielle Maßnahmenvermindert. Hierzu ist vorgeschlagen worden, zunächst das im CNSL vorhandene Cardol weitgehend selektiv mit Aldehyden, Aminen bzw. Basen und Hydroxiden von Alkali- und Erdalkalimetallen umzusetzen, und anschließend das nicht umgesetzte Cardanol abzudestillieren. Im Hinblick auf diese Verfahren zur Gewinnung von Cardanol mit verbesserter Farbstabilität sei auf die Dokumente GB-A-2,152,925, GB-A-2,066,820 und US-A-4,352,944 verwiesen.

GB 2066820 offenbart die Reinigung von CNSL.

### Beschreibung der Erfindung

Die Aufgabe der vorliegenden Erfindung bestand darin, ein Verfahren zur Herstellung von farbstabilem Cardanol bereitzustellen. Darüber hinaus sollten auch Folgeprodukte des nach diesem Verfahren hergestellten Cardanols, insbesondere Phenalkamine, farbstabil sein.

Überraschenderweise wurde gefunden, daß dies gelingt, indem rohes CNSL einer speziellen chemischen Behandlung nebst anschließender Destillation unterworfen wird. Vorzugsweise kann dann zusätzlich eine weitere Behandlung des Destillats mit chemischen Adsorbentien erfolgen.

Die essentiellen Verfahrensschritte des erfindungsgemäßen Verfahrens sind wie folgt:
1. Destillation, insbesondere Kurzwegdestillation, von rohem CNSL
2. Umsetzung des Destillats ("Roh-Cardanol") mit Borsäure
3. Destillation der Reaktionsmischung von Schritt 2, insbesondere Kurzwegdestillation

Der Hauptlauf von Schritt 3 kann gewünschtenfalls (optional) mit geringen Mengen Adsorbentien und/oder Reduktionsmitteln nachbehandelt werden. Dies ist eine optionale Maßnahme.

Gegenstand der Erfindung ist ein Verfahren zur Herstellung einer farbstabilem Zusammensetzung enthaltend Cardanol, wobei man
1. rohes CNSL (cashew nut shell liquid) zunächst einer Destillation, insbesondere einer Kurzwegdestillation, unterwirft,
2. das dabei erhaltene Destillat mit Borsäure umsetzt und
3. anschließend eine Destillation durchführt, wobei zunächst Leichtsieder abgetrennt werden und wobei der Hauptlauf das Zielprodukt (die farbstabile Cardanol/Cardol-Zusammensetzung) darstellt.

Das im Zuge des erfindungsgemäßen Verfahrens eingesetzte CNSL ist natürlichen Ur-sprungs - es wird wie bereits gesagt aus den äußeren Schalen von Cashewnüssen (Nüsse des Baumes Anacardium Occidentale) durch Extraktion gewonnen und kann hinsichtlich seiner Zusammensetzung variieren. Typischerweise enthält es 60-65% Cardanol, 2-10% Cardol, 10-15% Oligomere/Polymere und 0-2% 2-Methylcardanol und Anacardic acid.

Durch das erfindungsgemäße Verfahren wird eine farbstabile Zusammensetzung enthaltend Cardanol erhalten. Diese Zusammensetzung ist nicht nur als solche bei Lagerung farbstabil, sondern die auf Basis dieser Zusammensetzung hergestellten Phenalkamine sind es ebenfalls. Außerdem zeichnet sich die Zusammensetzung dadurch aus, dass sie im Vergleich zu den bisherigen handelsüblichen Produkten eine bessere Hautverträglichkeit aufweist, wodurch Handhabung und Transport sicherer werden.

In einer bevorzugten Ausführungsform wird der Hauptlauf von Schritt 3 des erfindungsgemäßen Verfahrens mit geringen Mengen Adsorbentien und/oder Reduktions-mitteln nachbehandelt. Durch den Einsatz von Adsorbentien und/oder Reduktionsmitteln wird eine weitere Erhöhung der Farbstabilität erzielt.

Zu den oben genannten drei obligatorischen Schritten des erfindungsgemäßen Verfahrens sei folgendes angemerkt:
- **Schritt 1** beinhaltet eine weitgehende Abtrennung von Oligomeren (mit Molgewichten im Bereich von 1000 bis 3000) im rohen CNSL. Die Abtrennung geschieht durch Destillation, wobei bereits eine einfache Kurzwegdestillation zu ausgezeichneten Ergebnissen führt. Bei dieser Abtrennung werden in der Regel etwa 15-20 Gew.-% des CNSL in Form der genannten Oligomeren entfernt. Die Kurzwegdestillation ist als Destillationsmethode bevorzugt. Sie ist mit einer raschen Prozessführung verbunden, wodurch Nebenprozesse wie Polymerisation oder Oxidation so weit als möglich unterbunden bzw. minimiert werden. Vorzugsweise wird die Kurzwegdestillation wie folgt durchgeführt: Man nimmt einen Vorlauf von 2 bis 5 Gew.-% (bezogen auf das eingesetzte CNSL) bei Temperaturen im Bereich von 150 bis 200 °C und Drucken im Bereich von 1 bis 5 mmHg und anschließend einen Hauptlauf bei Temperaturen im Bereich von 220 bis 260 °C und Drucken im Bereich von 1 bis 5 mmHg. Vorzugsweise setzt man eine Kurzweg-Destillationsapparatur ein, die mit einem Vorverdampfer ausgerüstet ist. Der Hauptlauf, das eigentliche Destillat aus Schritt 1, wird nachfolgend auch als RohCardanol bezeichnet.
- In **Schritt 2** wird das Destillat von Schritt 1 mit Borsäure (H₃BO₃) umgesetzt. Dadurch wird erreicht, dass die vorhandenen dihydrischen Phenole in die entsprechenden Borsäureester überführt werden. Vorzugsweise wird ein molares Verhältnis von Rohcardanol ex Schritt 1 zu Borsäure im Bereich von 3 : 0,07 bis 3 : 0,1 eingestellt. Die Reaktionstemperatur wird vorzugsweise auf Werte im Bereich von 120 bis 150 °C eingestellt. Die Reaktionszeit wählt man vorzugsweise im Bereich von 30 bis 90 Minuten, insbesondere etwa 1 Stunde. Gebildetes Reaktionswasser wird vorzugsweise kontinuierlich aus dem System entfernt (stripping off). Borsäure wird vorzugsweise in stöchiometrischer Menge in Bezug auf die im Rohcardanol ex Schritt 1 enthaltenen Cardole eingesetzt.
- Anschließend wird das Gemisch aus **Schritt 3** einer Destillation unterworfen, wobei die leichtsiedenden Bestandteile abgetrennt werden und die höhermolekularen Borsäureester im Rückstand verbleiben. Die Destillation kann dabei an sich nach allen dem Fachmann bekannten Methoden durchgeführt werden. In einer Ausführungsform arbeitet man im Vakuum unter Bedingungen einer Kurzwegdestillation oder einer konventionellen fraktionierenden Destillation. In einer Ausführungsform wird ein Vorlauf von etwa 2-5 Gew.% genommen, wobei man insbesondere bei 180 bis 210 °C und 1 bis 5 mmHg arbeitet. Die Hauptfraktion, die das gewünschte Zielprodukt im Sinne der Erfindung darstellt und die bei 220 bis 260 °C und 1 bis 5 mmHg anfällt, enthält etwa 80-90% des Gemisches aus Schritt 2 des Verfahrens. Im Rückstand verbleiben die genannten hochsiedenden Bestandteile.

Während die Schritte 1 bis 3 für das erfindungsgemäße Verfahren obligatorisch sind, ist **Schritt 4** optional. Hierbei werden noch vorhandene Verunreinigungen weitgehend entfernt. An sich besteht dabei keinerlei Beschränkungen hinsichtlich der Natur der eingesetzten Adsorbentien bzw. Reduktionsmittel. Beispiele geeigneter Reduktionsmittel sind etwa Nariumhydrosulfit (Na₂S₂O₄), Natriummetabisulfit (Na₂S₂O₅), Natriumborhydrid (NaBH₄), Lithiumaluminumhydrid (LiAlH₄), Zinnchlorid (SnCl2), or Magnesium Silicate. Als Adsorbentien kommen beispielsweise Magnesiumsilikat oder chemisch äquivalente Verbindungen zum Einsatz. Die Menge der Adsorbentien bzw. Reduktionsmittel kann gering gehalten werden. Vorzugsweise setzt man Mengen von 0,1 bis 5 Gew.-% (bezogen auf den in Schritt 3 erhaltenen Hauptlauf) ein, wobei Mengen um etwa 1 Gew.-% besonders bevorzugt sind.

Ein weiterer Offenbarungsgegenstand ist die Verwendung Cardanol-haliger Mischungen, erhältlich nach dem erfindungsgemäßen Verfahren, zur Herstellung farbstabiler Phenalkamine.

### Beispiele

### Beispiel 1: Erfindungsgemäßes Verfahren

Schritt 1: 1250 g/h CNSL (von Firma Resibras) wurden kontinuierlich in eine Kurzweg-Destillationsapparatur eingespeist, die mit einem Vorverdampfer ausgestattet war, wobei bei 240 °C / 1 mmHg bzw. 170 °C / 5 mmHg gefahren wurde. Bedingungen: Vorlauf im Vorverdampfer = 63 g/h; Hauptlauf = 940 g/h.

Schritt 2: 5000 g des Destillates aus dem Hauptlauf von Schritt 1 ("rohes Cardanol") wurden 1 Stunde lang bei 160 °C mit 5,9 g Borsäure umgesetzt. Bei der Veresterung entstehendes Wasser wurde dabei kontinuierlich aus dem System entfernt.

Schritt 3: Das Material aus Schritt 2 wurde kontinuierlich (940 g/h) einem Kurzweg-Destillationsapparat, der mit einem Vorverdampfer ausgestattet war, zugeführt, wobei bei 240 °C / 1 mmHg bzw. 180 °C / 5 mmHg gefahren wurde. Bedingungen: Vorlauf im Vorverdampfer = 60 g/h; Hauptlauf = 796 g/h. Das Destillat aus dem Vorverdampfer wurde in den Rohstoff von Schritt 1 zurückgeführt (Mischen mit CNSL).

Step 4: 796 g des Hauptlaufes von Schritt 3 wurden anschließend mit 7 g Magnesiumsilikat gemischt und die Mischung 30 Minuten bei 50 °C gerührt, anschließend wurde unter Einsatz eines Sparkler-Filters filtriert. Auf diese Weise wurde ein reines und farbstabiles Cardanol erhalten.

### Beispiel 2: Bestimmung der Farbstabilität

Das gemäß Beispiel 1 erhaltene Produkt wurde anschließend 60 Tage bei 20 °C gelagert. Dabei wurden von Zeit zu Zeit Proben genommen und deren Farbwerte (Gardner-Farbwerte) bestimmt. Ebenso wurde ein handelsübliches Cardanol dem Lagerungstest unterworfen und die farbwerte in gleicher Weise bestimmt. Die Ergebnisse sind Tabelle 1 zu entnehmen: Die Zeile "gemäß B1" enthält die Gardner-Farbwerte des erfindungsgemäßen Produktes gemäß Beispiel 1. Die Zeile "Standard" enthält die Gardner-Farbwerte eines handeslüblichen Cardanols (Cardanol von Firma Resibras).

**Tabelle 1**

| | 1 Tag | 7 Tage | 15 Tage | 30 Tage | 45 Tage | 60 Tage |
|---|---|---|---|---|---|---|
| gemäß B1 | 2,3 | 2,5 | 2,9 | 3,2 | 3,3 | 4,2 |
| Standard | 3 | 6 | 12 | 15 | 17 | nicht best. |

Es zeigt sich, dass das erfindungsgemäße Produkt dem handelsüblichen Standard bei weitem überlegen ist.

Darüber hinaus wurde festgestellt, dass Phenalkamine, die auf Basis des Produktes gemäß Beispiel 1, Aminen (insbesondere Diethyltetramin) und Formaldehyd hergestellt wurden, sich ebenfalls durch Farbstabilität auszeichneten.

## Patentansprüche

1. Verfahren zur Herstellung einer farbstabilem Zusammensetzung enthaltend Cardanol, wobei man in einem ersten Schritt rohes CNSL (cashew nut shell liquid) einer Destillation unterwirft, das dabei erhaltene Destillat in einem zweiten Schritt mit Borsäure umsetzt und das dabei erhaltene Reaktionsgemisch in einem dritten Schritt einer Destillation unterwirft.

2. Verfahren gemäß Anspruch 1, wobei man in Schritt 1 eine Kurzwegdestillation durchführt.

3. Verfahren gemäß Anspruch 2, wobei man bei der Kurzwegdestillation einen Vorlauf von 1 bis 5 Gew.-% nimmt.

4. Verfahren gemäß einem der Ansprüche 1 bis 3, wobei man in Schritt 2 Borsäure in stöchiometrischer Menge in Bezug auf die im Rohcardanol ex Schritt 1 enthaltenen Cardole einsetzt.

5. Verfahren gemäß Anspruch 4, wobei man gebildetes Reaktionswasser kontinuierlich aus dem System entfernt.

6. Verfahren gemäß einem der Ansprüche 1 bis 5, wobei man in Schritt 3 eine Kurzwegdestillation durchführt..

7. Verfahren gemäß Anspruch 6, wobei man bei der Kurzwegdestillation in Schritt 3 einen Vorlauf von 1 bis 5 Gew.-% nimmt.

8. Verfahren gemäß einem der Ansprüche 1 bis 7, wobei man das erhaltene Produkt zusätzlich einer Nachbehandlung durch Adsorbentien und/oder Reduktionsmittel unterzieht.

9. Verfahren gemäß Anspruch 9, wobei man im Anschluß an die Nachbehandlung eine Filtration durchführt.

## Claims

1. A process for the production of a color-stable composition containing cardanol, wherein in a first step crude cashew nut shell liquid (CNSL) is subjected to distillation, in a second step the distillate obtained is reacted with boric acid, and in a third step the reaction mixture obtained is subjected to distillation.

2. The process according to claim 1, wherein a short-path distillation is carried out in step 1.

3. The process according to claim 2, wherein a first fraction of 1 to 5% by weight is taken in the short-path distillation.

4. The process according to any of claims 1 to 3, wherein boric acid is used in a stoichiometric amount in relation to the cardols present in the crude cardanol from step 1.

5. The process according to claim 4, wherein water of reaction formed is continuously removed from the system.

6. The process according to any of claims 1 to 5, wherein a short-path distillation is carried out in step 3.

7. The process according to claim 6, wherein a first fraction of 1 to 5% by weight is taken in the short-path distillation in step 3.

8. The process according to any of claims 1 to 7, wherein the product obtained is additionally subjected to aftertreatment by adsorbents and/or reducing agents.

9. The process according to claim 9, wherein the aftertreatment is followed by filtration.

## Revendications

1. Procédé pour la préparation d'une composition de couleur stable, contenant du cardanol, du baume de cajou (cashew nut shell liquid - CNSL) brut étant soumis à une distillation dans une première étape, le distillat obtenu étant transformé avec de l'acide borique dans une deuxième étape et le mélange réactionnel ainsi obtenu étant soumis à une distillation dans une troisième étape.

2. Procédé selon la revendication 1, une distillation instantanée étant réalisée dans l'étape 1.

3. Procédé selon la revendication 2, une première fraction de 1 à 5% en poids étant prélevée lors la distillation instantanée.

4. Procédé selon l'une quelconque des revendications 1 à 3, l'acide borique étant utilisé, dans l'étape 2, en des quantités stoechiométriques par rapport aux cardols contenus dans le cardanol brut de l'étape 1.

5. Procédé selon la revendication 4, l'eau de réaction formée étant éliminée en continu du système.

6. Procédé selon l'une quelconque des revendications 1 à 5, une distillation instantanée étant réalisée dans l'étape 3.

7. Procédé selon la revendication 6, une première fraction de 1 à 5% en poids étant prélevée lors la distillation instantanée dans l'étape 3.

8. Procédé selon l'une quelconque des revendications 1 à 7, le produit obtenu étant, en plus, soumis à un post-traitement par des adsorbants et/ou des réducteurs.

9. Procédé selon la revendication 9, une filtration étant réalisée après le post-traitement.
